# EUROPÄISCHE PATENTSCHRIFT

(11) **EP 2 621 413 B1**
(45) Veröffentlichungstag und Bekanntmachung des Hinweises auf die Patenterteilung: **11.06.2014**
(21) Anmeldenummer: 11760713.5
(22) Anmeldetag: 23.09.2011
(51) Int. Cl.: A61F 2/64, F16C 11/00, A61F 2/00, A61F 2/50, A61F 2/68, A61F 2/74, A61F 5/01

(54) **GELENKEINRICHTUNG**
JOINT MECHANISM
DISPOSITIF D'ARTICULATION

(30) Priorität: 28.09.2010 DE 102010046690
(43) Veröffentlichungstag der Anmeldung: 07.08.2013
(73) Patentinhaber: Otto Bock Healthcare Products GmbH, 1070 Wien (AT)
(72) Erfinder: DIETL, Hans, A-3003 Gablitz (AT); BRÖCKL, Heinz, A-1170 Wien (AT)
(74) Vertreter: Stornebel, Kai
(86) Internationale Anmeldenummer: PCT/EP2011/004772
(87) Internationale Veröffentlichungsnummer: WO 2012/041463

(56) Entgegenhaltungen:
- EP-A1- 0 016 268
- WO-A1-2009/110097
- DE-A1- 3 414 869
- DE-A1- 10 311 189
- DE-A1- 10 351 916
- DE-U1-202006 007 461
- GB-A- 691 264
- US-A1- 2005 149 203

## Beschreibung

Die Erfindung betrifft eine Gelenkeinrichtung einer Knieorthese oder einer Knieprothese mit einem ersten Teil und einem relativ dazu verschwenkbaren zweiten Teil und Anschlussmitteln zum Befestigen der Gelenkeinrichtung an einem Nutzer der Knieorthese oder Knieprothese mit einer Verriegelungseinrichtung, mit der eine Verschwenkung der beiden Teile zueinander in Flexionsrichtung durch ein an dem zweiten Teil verlagerbar angeordnetes Formschlusselement verhindert wird, dass im Verriegelungszustand formschlüssig in eine dem ersten zugeordnete Ausnehmung eingreift.

Kniegelenke, entweder für Orthesen oder Prothesen, die in einer bestimmten Stellung verriegelbar sind, werden als Sperrkniegelenke bezeichnet. Es gibt verschiedene Ausführungsformen von Sperrkniegelenken, die überwiegend dem Zweck dienen, dem Nutzer der Kniegelenke eine möglichst hohe Sicherheit zu geben. Insbesondere bei Prothesenträgern handelt es sich um eine Patientengruppe in der eher inaktive, meist geriatrische Patienten vorhanden sind, denen beim Gehen und Stehen durch ein in gestreckter Position gesperrtes Kniegelenk ein erhöhtes Sicherheitsgefühl vermittelt wird. Diese Gruppe von Prothesennutzern ist nicht in der Lage, ein plötzliches, ungewolltes Einbeugen des Gelenks durch Ausgleichsbewegungen zu verhindern. Auch für Orthesennutzer kann eine Verriegelung in der gestreckten Stellung sinnvoll sein, wenn die vorhandene Muskulatur nicht ausreicht, um in bestimmten Situationen das Bein stabil zu halten.

Die DE 103 51 916 A1 zeigt ein Prothesenkniegelenk mit einem Oberteil und einem über eine mehrachsige Gelenkeinrichtung daran befestigten Unterteil. Das Unterteil ist in Streckrichtung stets ungehindert verschwenkbar, ein Verriegelungselement ist vorgesehen, das ein Nebenventil einer hydraulischen Dämpfereinheit in der geöffneten Stellung hält.

Die DE 20 2006 007 461 U1 beschreibt ein Prothesenkniegelenk mit einem Oberteil und einem gelenkig daran befestigten Unterteil. Zwischen dem Oberteil und dem Unterteil ist eine ver- und entriegelbare Sperreinrichtung vorgesehen, die im verriegelten Zustand eine Verschwenkung des Oberteils zu dem Unterteil verhindert.

Die DE 103 11 189 A1 beschreibt ein orthopädietechnisches Hilfsmittel mit relativ zueinander bewegbaren Teilen und einer Verriegelungsvorrichtung zum Verriegeln der beiden Teil in einer vorbestimmten relativen Position und zum Entriegeln der Teile zur Freigabe der Bewegung der Teile zueinander. Das orthopädietechnische Hilfsmittel ist insbesondere als eine Orthese ausgebildet. Ein Endabschnitt eines Gelenkunterteils ist mit einer radialen Ausnehmung versehen, in die ein Verriegelungsstift, der an dem Gelenkoberteil gelagert ist, mit einem unteren, komplementär zu der Ausnehmung geformten Ende eingreift. Der Verriegelungsstift geht an seinem oberen Ende in einen zylindrischen Kern über, der in einem Innenraum einer elektrischen Spule axial bewegbar ist. Wird Strom durch die Spule geleitet, wird der Verriegelungsstift nach oben in den Spuleninnenraum gezogen.

Die GB 691,246 betrifft ein Kniegelenk für künstliche Beine mit einem Oberschenkelteil, einem Unterschenkelteil und einem Fußabschnitt. Der Oberschenkelteil und der Unterschenkelteil sind gelenkig miteinander verbunden und gleiten auf Parabelförmigen Gleitflächen aufeinander. Ein Reibelement ist vorgesehen, um in Abhängigkeit von dem aufgebrachten Gewicht ein Einbeugen zu ermöglichen oder zu verhindern,

Die EP 0 016 268 A1 betrifft ein verriegelbaren Orthesengelenk mit zwei gelenkig zueinander gelagerten Komponenten. Eine Komponente weist einen Schlitz auf, in den ein vorgespanntes Verriegelungselement eingreift, wenn die beiden Komponenten eine entsprechend zueinander ausgerichtete Stellung aufweisen. Ein Halteelement hält das Verriegelungselement in der geöffneten Stellung.

Die DE 34 14 869 A1 betrifft ein künstliches Kniegelenk mit einem oberen und einem unteren Gelenkteil mit einem federbelasteten Sperrnocken, der in einem Unterschenkelteil längsbeweglich angeordnet ist. An dem Sperrbolzen greift ein in dem unteren Gelenkteil gelagerter, doppelarmiger Hebel an. An dem Oberschenkelteil ist ein Kurvenstück angeordnet, dessen gekrümmte Umfangsfläche eine Stirnfläche des Sperrbolzens bei gelöster Sperre anliegt und gegen dessen mit der Umfangsfläche einen winkeleinschließenden Stirnfläche der Sperrbolzen in der Sperrstellung anliegt,

Die US 2005/0149203 A1 betrifft ein künstliches Prothesenkniegelenk mit einem Verriegelungsinechanismus, der das Prothesenkniegelenk in einer gestreckten Stellung hält. In Abhängigkeit von einem ermittelten Kraftverlauf wird die Verriegelungseinrichtung über eine Steuereinrichtung entriegelt. Die Verriegelung findet über einen Schwenkhebel statt, der einen hakenförmigen Bereich aufweist, der in einen korrespondierend geformten Absatz im Oberteil des Prothesenkniegelenkes eingreift.

Die WO 2009/110097 A1 betrifft ein Prothesenkniegelenk mit einem manuellen Verriegelungsmechanismus, um ein Oberteil eines Prothesenkniegelenkes mit einem Unterteil zu verriegeln. Der manuelle Verriegelungsmechanismus beinhaltet eine oszillierende Welle mit einem unregelmäßig geformten Teil und einem Verriegelungsteil zum Regeln der Bewegung der Welle. Zum Entriegeln wird das Verriegelungsteil außer Eingriff mit dem unregelmäßig geformten Teil der oszillierenden Welle gebracht.

Aufgabe der vorliegenden Erfindung ist es, eine Gelenkeinrichtung bereitzustellen, die auch für aktive Prothesengeher geeignet ist, aber zusätzlich vom Nutzer auch im gestreckten Zustand verriegelt werden kann, um eine volle Belastbarkeit in besonderen Situationen zu gewährleisten und insbesondere ein sicheres Gehen und Stehen in Nassbereichen zu ermöglichen.

Erfindungsgemäß wird diese Aufgabe durch eine Vorrichtung mit den Merkmalen des Hauptanspruches gelöst. Vorteilhafte Ausgestaltungen und Weiterbildungen der Erfindung sind in den Unteransprüchen, der Beschreibung sowie in den Figuren aufgeführt. Die erfindungsgemäße Gelenkeinrichtung einer Knieorthese oder Knieprothese mit einem ersten Teil und einem relativ dazu verschwenkbaren zweiten Teil und Anschlussmitteln zum Befestigen der Gelenkeinrichtung an einem Nutzer der Knieorthese oder Knieprothese sowie eine Verriegelungseinrichtung, mit der eine Verschwenkung der beiden Teile zueinander in Flexionsrichtung durch ein an dem zweiten Teil verlagerbar angeordnetes Formschlusselement verhindert wird, das im Verriegelungszustand formschlüssig in eine dem ersten Teil zugeordnete Ausnehmung eingreift, sieht vor, dass das Formschlusselement drehbar an dem zweiten Teil gelagert ist und eine Kontur aufweist, die in der Verriegelungsstellung in die Ausnehmung hineinragt und in der Freigabestellung ein Vorbeischwenken des ersten Teils an der Lagerungsstelle des Formschlusselementes ermöglicht. Die Verriegelungseinrichtung mit dem Formschlusselement und der Ausnehmung bewirkt eine Sperrung des Kniegelenks in Flexionsrichtung über eine Blockierung der Relativbewegung zwischen dem ersten Teil und dem zweiten Teil, üblicherweise dem Unterteil und dem Oberteil. Grundsätzlich ist der Ort der Anordnung des Formschlusselementes und der Ausnehmung frei wählbar, aufgrund der einfacheren Zuordnung und Bedienbarkeit wird das Formschlusselement in der Regel an dem Unterteil der Gelenkeinrichtung angeordnet sein, also an dem Knierahmen, während die Ausnehmung an dem Oberteil oder einem Kniekopf angeordnet ist. Im gesperrten Zustand der Gelenkeinrichtung, also in der Verriegelungsstellung des Formschlusselements, wird die Bewegung zwischen dem Kniekopf und dem Knierahmen mit Hilfe des drehbar gelagerten Formschlusselementes verhindert. Soll das Kniegelenk wieder frei in Flexionsrichtung gebeugt werden können, muss es durch eine Drehung des Formschlusselementes entriegelt werden. Dadurch wird die Kontur, die nicht rotationssymmetrisch ausgebildet ist, in eine Stellung bewegt, in der kein formschlüssiger Eingriff des Formschlusselementes in die Ausnehmung, die an dem Formschlusselement vorbeischwenkt gegeben ist. Das Formschlusselement ist als Bolzen mit einem runden oder ovalen Querschnitt mit einer Abflachungsausbildung eines Durchganges für den ersten Teil ausgebildet. Die Abflachung an dem Bolzen kann durch Abschleifen, Abfräsen oder durch das Vorsehen einer Ausnehmung, die gerade, gerundet oder speziell angepasst sein, an dem Bolzen ausgebildet sein. Die Abflachung von dem Bolzen ermöglicht dann die freie Bewegung des ersten Teils relativ zu dem zweiten Teil, indem das erste Teil in dem Bereich der Abflachung an dem Bolzen vorbeischwenkt. Bei einer ovalen Querschnittsform des Bolzens schwenkt das zweite Teil in der Freigabestellung an der Kontur mit den kurzen Achsen vorbei, in der Verriegelungsstellung greift die Kontur mit der langen Achse in die Ausnehmung ein.

Das Formschlusselement kann in Richtung auf die Verriegelungsstellung federbelastet vorgespannt sein. Dadurch ist es möglich, dass das Formschlusselement automatisch in die Ausnehmung einrastet, wenn das erste Teil und das zweite Teil der Gelenkeinrichtung so zueinander orientiert sind, dass die Verriegelungsstellung erreicht ist. Befindet sich das Formschlusselement in der Freigabestellung wenn sich die beiden Teile der Gelenkeinrichtung nicht in der Verriegelungsstellung befinden, ist eine Flexionsbewegung und Extensionsbewegung ohne weiteres möglich. Bedient der Nutzer der Gelenkeinrichtung die Verriegelungseinrichtung beispielsweise im gebeugten Zustand des Knies und kommt es anschließend zu einer Streckung des Gelenks in Extensionsrichtung, rastet das Formschlusselement aufgrund der Federvorspannung automatisch in die Ausnehmung ein und verriegelt das zweite Teil gegen eine Relativbewegung zu dem ersten Teil.

Es kann zudem vorgesehen sein, dass das Formschlusselement exzentrisch gelagert ist und durch eine Drehung um die Schwenkachse aus der Ausnehmung herausgeschwenkt wird, um eine Freigabestellung einzunehmen, oder wieder hereingeschwenkt wird, um eine Verriegelungsstellung einzunehmen.

Die Gelenkeinrichtung ist vorzugsweise als eine monozentrische Gelenkeinrichtung ausgebildet, vorzugsweise ist die Gelenkeinrichtung auch wasserbeständig und ggf, wasserdicht gegenüber der Umgebung abgedichtet. Durch die monozentrische Ausgestaltung der Gelenkeinrichtung ist es relativ einfach aufgebaut, so dass eine Robustheit des Kniegelenkt bereitgestellt wird, mit der der Nutzer eine Vielzahl an Aktivitäten durchführen kann. Auch monozentrische Gelenkeinrichtungen, beispielsweise mit einer hydraulischen oder pneumatischen Dämpfereinrichtung, die zur Steuerung der Schwungphase verwendet wird, eignen sich für aktive Geher und aufgrund der robusten und einfachen Bauweise auch für hohe Beanspruchungen. Daher ist es möglich, dass trotz der einfachen Ausführungsform des Gelenks bei annährend gleichen Funktionen im Vergleich zu Gelenken mit einer elektronischen Steuerung eine erhöhte Unabhängigkeit und darüber hinaus eine erhöhte Zuverlässigkeit gewährleistet wird.

Um eine ungewollte Verriegelung oder Entriegelung der Gelenkeinrichtung zu verhindern, ist das Formschlusselement gegen eine Verdrehung gesichert an dem zweiten Teil gelagert, so dass erst eine Sicherung überwunden werden muss, um eine Entriegelung oder Verriegelung vorzunehmen.

Eine federbelastete Fixiereinrichtung kann eine formschlüssige Sicherung des Formschlusselementes ausbilden. Alternative Fixiereinrichtungen sind möglich und vorgesehen, beispielsweise eine elektromagnetische oder motorische Kraftbeaufschlagung der Fixiereinrichtung. Erst durch eine bewusste Entfernung der Fixiereinrichtung aus der Fixierstellung ist es möglich, das Formschlusselement aus der Verriegelungsstellung in die Freigabestellung zu bewegen und umgekehrt. Der Fixiereinrichtung ebenso wie dem Formschlusselement kann eine Einrichtung zur Vermittlung eines Haptik-Feedbacks zugeordnet sein, beispielsweise eine Kurvenbahn entlang der das Formschlusselement bewegt werden muss, so dass eine Rastung oder ein Bewegungswiderstand überwunden werden muss. Das Einrasten in die jeweilige Endstellung ergibt dann einen mechanischen Impuls, der von dem Nutzer als haptische Rückkopplung erkannt wird, dass sich das Formschlusselement oder Fixiereinrichtung in der einen oder anderen Stellung befindet.

Zwischen dem ersten Teil und dem zweiten Teil der Gelenkeinrichtung ist vorzugsweise ein Dämpfer, insbesondere ein hydraulischer Dämpfer angeordnet, der neben einer Schwungphasensteuerung als Ergebnis der Bewegungswiderstände auch eine Beugeendlagendämpfung und eine Streckanschlagdämpfung bereitstellt. Die Schwungphasesteuerung ergibt sich während der Schwungphase des Gehens durch eine entsprechend ausgebildete Dämpfungscharakteristik. Diese Charakteristik ist dabei so gestaltet, dass für den jeweiligen Nutzer ein harmonisches Gangbild erreicht wird. Teil des harmonischen Gangbildes ist eine Streckanschlagdämpfung sowie eine Beugeendlagendämpfung, die durch eine Ausgestaltung von Strömungskanälen innerhalb des Dämpfers erreicht werden kann. Ist der Dämpfer als Hydraulikdämpfer ausgebildet, erfolgt der Abfluss des durch den Kolbenhub verdrängten Hydrauliköls beispielsweise durch eine Bohrung in der Kolbenstange. In diese Bohrung mündet ein schmaler, z.B. gewundener Kanal, der in die Oberfläche der Kolbenstange eingearbeitet ist. Sobald der Kolben die Bohrung verschließt und das Öl über den Kanal abfließen muss, kommt es durch die Drosselwirkung und eine speziell ausgebildete Geometrie des Kanals zu einem sukzessiven Anstieg der Dämpfung. Je größer die Verringerung des Strömungsquerschnittes ist, desto größer ist der Anstieg der Dämpfung. Für die Flexionsseite und Extensionsseite können unterschiedliche Kanalgeometrien ausgebildet sein, um eine unterschiedliche Dämpfung in Flexionsrichtung und Extensionsrichtung zu erreichen.

Dem Formschlusselement können Drehanschläge zugeordnet sein, so dass beispielsweise bei einer ovalen Ausgestaltung des Formschlusselementes eine Drehbarkeit nur um einen festgelegten Maximal-Drehwinkel erfolgen kann, beispielsweise 90° oder 180°. Je nach Ausgestaltung der Abflachung oder Ausnehmung in dem z.B. als Bolzen ausgestalteten Formschlusselement kann auch bei einem ansonsten runden Querschnitt des Formschlusselementes eine Drehebereichsbegrenzung für das Formschlusselement vorgesehen sein.

Der Fixiereinrichtung, um das Formschlusselement gegen ein unbeabsichtigtes Betätigen zu sichern, kann ein fernbedienbarer Aktuator zugeordnet sein, der sowohl die Fixierung aufhebt als auch das Formschlusselement aus der Verriegelungsstellung in die Freigabestellung und gegebenenfalls zurückbewegt.
Nachfolgend werden Ausführungsbeispiele der Erfindung anhand der beigefügten Figuren näher erläutert. Es zeigen:
- Figur 1 -: eine schematische Darstellung eines Prothesenkniegelenkes in Seitenansicht in Verriegelungsstellung';
- Figur 2 -: ein Prothesenkniegelenk in Freigabestellung;
- Figur 3 -: ein Formschlusselement als Bolzen;
- Figur 4 -: eine Schnittdarstellung des Formschlusselementes;
- Figur 5 -: eine Detaildarstellung einer Fixiereinrichtung;
- Figur 6 -: zwei Darstellungen einer Variante des Prothesenkniegelenks; sowie
- Figur 7 -: zwei Detaildarstellungen der Figur 6.

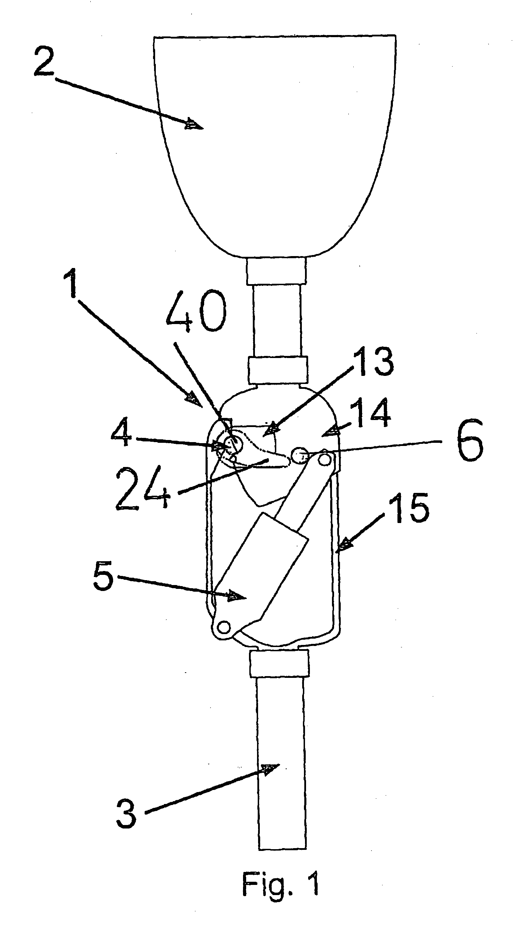

In der Figur 1 ist eine schematische Darstellung eines Prothesenkniegelenkes 1 in einer Seitenansicht mit einem Oberschenkelschaft 2 zur Festlegung an einem Kniegelenksnutzer sowie einem Unterschenkelrohr 3 dargestellt. Der Oberschenkelschaft 2 dient zur Aufnahme eines Oberschenkelstumpfes, das Unterschenkelrohr 3 dient zur Aufnahme eines nicht dargestellten Prothesenfußes. Zwischen dem Oberschenkelschaft 2 und dem Unterschenkelrohr 3 ist das Prothesenkniegelenk 1 angeordnet, das ein erstes Teil 14 und ein schwenkbar daran gelagertes zweites Teil 15 aufweist. Das erste Teil 14 wird üblicherweise als Oberteil des Prothesenkniegelenkes 1 bezeichnet, das zweite Teil 15 üblicherweise als das Unterteil. Das Prothesenkniegelenk 1 in der dargestellten Ausführungsform ist als monozentrisches Kniegelenk ausgestaltet, das zweite Teil 15, das auch als Kniegelenksrahmen bezeichnet werden kann, verschwenkt relativ zu dem ersten Teil 14, das auch als Kniegelenkskopf bezeichnet wird, um eine Schwenkachse 6. An dem ersten Teil 14 ist dorsal zu der Schwenkachse 6 eine hydraulische Dämpfereinrichtung 5 in Gestalt eines Hydraulikdämpfers angeordnet, der Lagerungspunkt an dem zweiten Teil 15 liegt ventral zu der Schwenkachse 6. An dem zweiten Teil 15 ist darüber hinaus ein Formschlusselement 4 angeordnet, das als drehbarer Bolzen ausgebildet ist. In der Figur 1 ist das Prothesenkniegelenk 1 in einer Verriegelungsstellung gezeigt, der drehbarer Bolzen 4 greift in eine Ausnehmung 40 ein, die an dem ersten Teil 14 ausgebildet ist. Die Ausnehmung 40 befindet sich an einer Außenkontur des ersten Teils 14, der an dem Formschlusselement 4 vorbeischwenkt, wenn das Prothesenkniegelenk 1 gebeugt wird. Im dargestellten verriegelten Zustand sperrt das als Bolzen ausgebildete Formschlusselement 4 eine Beugebewegung zwischen dem ersten Teil 14 und dem zweiten Teil 15. Grundsätzlich ist es auch möglich, das Formschlusselement 4 an dem ersten Teil 14 anzuordnen, während die Ausnehmung 40 an dem zweiten Teil 15 ausgebildet ist.

Weiterhin ist in der Figur 1 eine Fixiereinrichtung 13 zu erkennen, die an dem Formschlusselement 4 angeordnet ist. Die Fixiereinrichtung 13 dient zur Umschaltung des Formschlusselementes 4 sowie zur Aktivierung oder Deaktivierung der mechanischen Sperre des Prothesenkniegelenks 1. Sowohl der Aufbau als auch die Funktionsweise der Fixiereinrichtung 13 wird später näher erläutert. Über einen Hebel 24 kann das Formschlusselement 4 aus der dargestellten Verriegelungsstellung in eine Freigabestellung und umgekehrt verschwenkt werden. Die Verriegelung findet dabei in der gestreckten Stellung statt, das Formschlusselement 4 wird in die Ausnehmung 40 eingeschwenkt und bewirkt eine formschlüssige Sperrung.

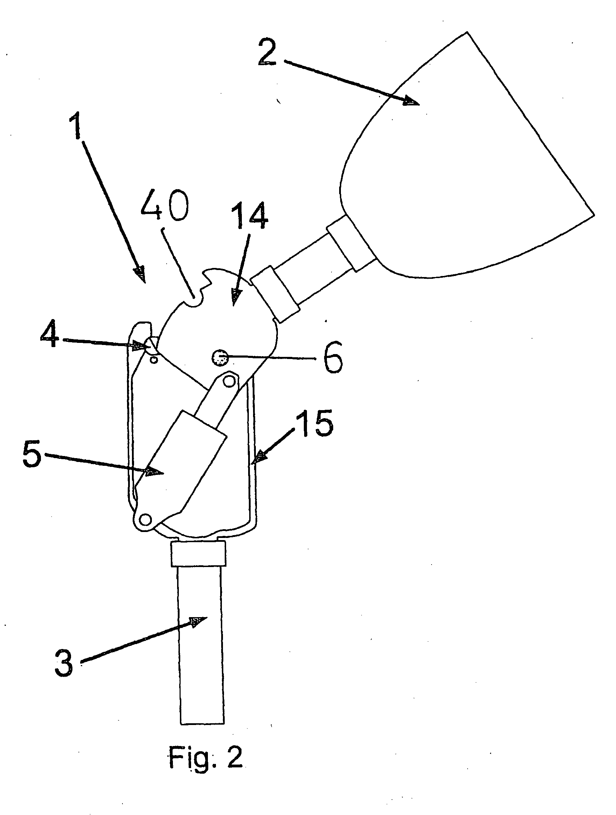

In der Figur 2 ist das Prothesenkniegelenk 1 in einer gebeugten Stellung gezeigt. Der Oberschenkelschaft 2 und das Unterschenkelrohr 3 sind relativ zueinander um die Schwenkachse 6 verlagert, der Hydraulikdämpfer 5 ist gegenüber der Verriegelungsstellung gemäß Figur 1 verkürzt. An dem ersten Teil 14 ist die Ausnehmung 40 zu erkennen, die im dargestellten Ausführungsbeispiel eine halbkreisförmige Kontur aufweist. Diese Ausnehmung 40 ist in die Kontur des ersten Teils 14 eingearbeitet, die beim Einbeugen des Prothesenkniegelenkes 1 an dem Formschlusselement 4 vorbei schwenkt. Das Formschlusselement 4 weist eine asymmetrische Kontur auf, im dargestellten Ausführungsbeispiel ist derjenige Bereich des Formschlusselementes 4, der für die Verriegelung zuständig ist, als ein Halbkreis ausgebildet. Liegt diejenige Hälfte des Halbkreises ohne Material der Ausnehmung 40 gegenüber, befindet sich das Formschlusselement in einer Freigabestellung, ragt der massive Teil des Formschlusselementes 4 in die Ausnehmung 40 hinein, entweder vollständig oder zumindest teilweise, ist das Prothesenkniegelenk 1 gegen eine Beugung gesperrt. Durch die Halbkreisform sowohl des Formschlusselementes 4 als auch der Ausnehmung 40 und der Verriegelung in der gestreckten Stellung ist es nicht notwendig, dass das Formschlusselement 4 vollständig in der Ausnehmung einrastet, vielmehr reicht schon eine relative geringe Überdeckung und ein Hineinragen des massiven Teils aus, um eine Sperrung zu bewirken.

Sobald sich das Formschlusselement 4 aus der Verriegelungsstellung in die Freigabestellung bewegt hat und das erste Teil 14 gegenüber dem zweiten Teil 15 so um die Schwenkachse 6 verschwenkt wurde, dass die Ausnehmung 40 soweit von dem Formschlusselement 4 weg verlagert wurde, dass der massive Teil des Formschlusselementes 4 nicht mehr in die Ausnehmung 40 eingreifen kann, kann das Prothesenkniegelenk 1 stets gebeugt und bis zur erneuten Verriegelung gestreckt werden.

Das Formschlusselement 4 kann dabei mit einer Vorspannung, insbesondere einer Federvorspannung versehen sein, die das Formschlusselement 4 in eine Verriegelungsstellung drängt. In der Figur 2 ist die Stellung gezeigt, bei der das erste Teil 14 soweit verschwenkt wurde, dass das Formschlusselement 4 nicht mehr an der leicht gebogenen Außenkontur unterhalb der Ausnehmung 40 entlang gleitet. Das Formschlusselement 4 befindet sich an der Unterkante des ersten Teils 14 in einer Stellung, in der es in die Ausnehmung 40 eingreifen könnte. Eine weitergehende Beugung ist ohne weiteres möglich. Wird das Prothesenkniegelenk 1, ausgehend von der Stellung der Figur 2, gestreckt, tritt die Unterseite des ersten Teils 14 dergestalt mit dem Formschlusselement 4 in Kontakt, dass es in eine Freigabestellung verschwenkt wird. Das Formschlusselement 4 gleitet dann bei einer weiteren Streckung solange auf der dem Formschlusselement 4 zugewandten Kontur des ersten Teils 14 entlang, bis die Ausnehmung 40 erreicht ist. Aufgrund der Federvorspannung schwenkt das Formschlusselement 4 dann automatisch in die Verriegelungsstellung hinein und verriegelt das Prothesenkniegelenk 1 in der gestreckten Stellung, wie es in der Figur 1 dargestellt ist.

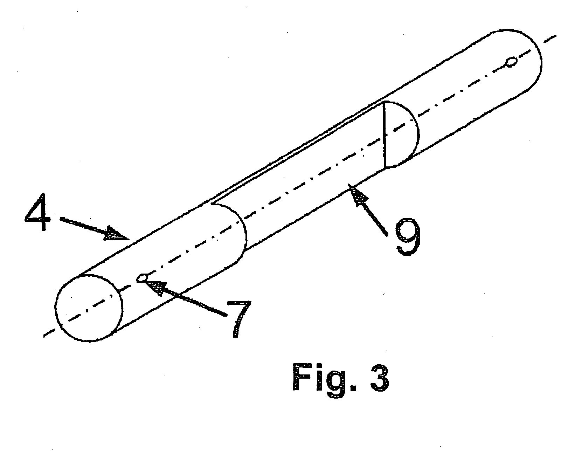

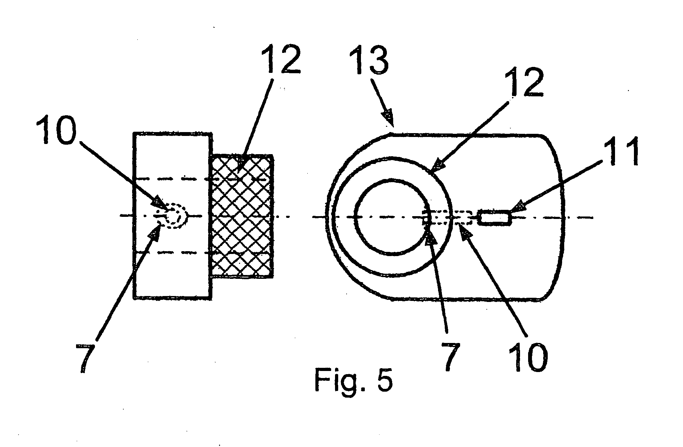

In der Figur 3 ist das Formschlusselement 4 in einer Detaildarstellung gezeigt. Das Formschlusselement 4 ist als ein zylindrischer Bolzen mit einer mittig angeordneten, erfindungsgemäßen Abflachung 9 ausgebildet. Die Abflachung 9 dient zur Freigabe einer Relativbewegung zwischen dem ersten Teil 14 und dem zweiten Teil 15. Durch die Abflachung 9 wird Raum für das Vorbeischwenken bzw. Durchschwenken des ersten Teils durch die Abflachung 9 hindurch bereitgestellt, so dass das Prothesenkniegelenk 1 gebeugt werden kann. An zumindest einem Endbereich des Formschlusselementes 4 ist eine Ausnehmung 7 zur Aufnahme eines Fixierstiftes ausgebildet. Über den Fixierstift, der in der Figur 5 näher erläutert werden wird, wird das Formschlusselement 4 gegen eine unbeabsichtigte Verdrehung geschützt.

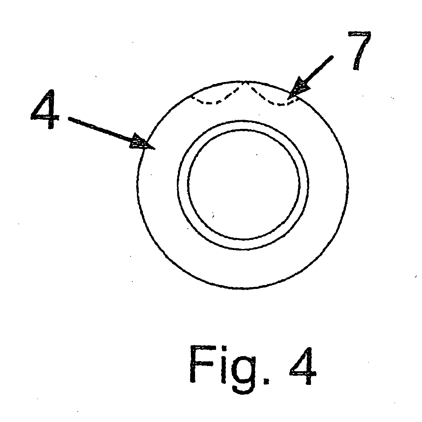

In der Figur 4 ist eine Schnittdarstellung gezeigt, in der zwei Ausnehmungen 7 für den Fixierstift angedeutet sind, die eine Ausnehmung 7 fixiert das Formschlusselement 4 in der Verriegelungsstellung, die andere in der Freigabestellung.

Die Figur 5 zeigt zwei Ansichten der Fixiereinrichtung 13. An der Fixiereinrichtung 13 ist eine Vorrichtung 12 zur Umschaltung bzw. zur Aktivierung oder Deaktivierung einer mechanischen Sperre angeordnet. Die Vorrichtung 12 schaltet zwischen den Zuständen "gesperrt" und "ungesperrt" um. Dabei ist in der Vorrichtung 12 ein Aktuator 11 vorgesehen, über den ein Fixierstift 10 in die Ausnehmung 7 hineinbewegt bzw. aus der Ausnehmung 7 herausbewegt werden kann. Der Aktuator 11 dient zur Beaufschlagung des Fixierstiftes 10 mit einer Zwangskraft, die entweder den Fixierstift 10 aus der Ausnehmung 7 herausbewegt oder in die Ausnehmung 7 innerhalb des Formschlusselementes 4 hineindrückt.

Der dargestellte Verriegelungs- und Entriegelungsmechanismus mit dem Formschlusselement 4 ist ähnlich einem Ziehkeil, wie er in einem mechanischen Getriebe verwendet wird. Dabei kann das Formschlusselement 4 beispielsweise über den Hebel 24, wie er in der Figur 1 gezeigt ist, entweder von Hand oder über einen Aktuator fernbedienbar ausgelöst werden. Andere Betätigungselemente können ebenfalls vorgesehen sein, beispielsweise eine Rändelkappe oder ein Motor. Im dargestellten Ausführungsbeispiel wird der Hebel 24 um die Schwenkachse des Formschlusselementes 4 nach unten geschwenkt, so dass das erste Teil 14 des Prothesenkniegelenkes 1 durch die Abflachung 9 hindurchschwenken kann. Im gesperrten Zustand wird die Bewegung zwischen dem ersten Teil 14 und dem zweiten Teil 15 durch den massiven Teil des halbierten Bolzens verhindert. Die Sperrung des Prothesenkniegelenkes 1 kann nur in gestreckter Kniestellung erfolgen, da nur in dieser Stellung eine Relativbewegung zwischen dem ersten Teil 14 und dem zweiten Teil 15 durch einen formschlüssigen Eingriff des massiven Teils des Formschlusselementes 4 in die Ausnehmung 40 verhindert werden kann.

Das Formschlusselement 4 kann in der gewünschten Stellung durch die Fixiereinrichtung 13 festgelegt werden, vorzugsweise über den Fixierstift 10, der mittels Federkraft, Kabelzug oder motorisch in eine entsprechende Ausnehmung 7 innerhalb des Formschlusselementes 4 gedrückt wird und dadurch eine Verdrehung des Formschlusselementes 4 relativ zu dem zweiten Teil 15, also seiner Lagerstelle, verhindert. Die Überwindung der Federkraft, also die Entsperrung des Formschlusselementes 4 erfolgt bevorzugt von Hand oder ebenfalls mittels einer Fernbedienung, so dass dann das Formschlusselement 4 zwischen der offenen und der gesperrten Stellung umgeschaltet werden kann.

Bedient der Prothesenträger den Verriegelungsmechanismus bei gebeugtem Knie, bewegt sich das Formschlusselement 4 in die Verriegelungsstellung, das Prothesenkniegelenk 1 bleibt jedoch weiterhin in Flexionsrichtung frei beweglich. Bei einer Streckung des Prothesenkniegelenkes wird das Formschlusselement 4 wieder in die Freigabestellung gedreht, in der das Gelenk ungesperrt ist. Sofern keine dauerhafte Beaufschlagung des Formschlusselementes 4 in Richtung auf eine Verriegelungsstellung vorhanden ist, bleibt dann das Prothesenkniegelenk 1 ungesperrt. Ist eine Vorspannung oder Vorbelastung in Richtung auf die Verriegelungsstellung gegeben, rastet das Formschlusselement automatisch bei Erreichen einer gestreckten Stellung in die Ausnehmung 40 ein und verriegelt das Prothesenkniegelenk 1.

Bei der Betätigung der Fixiereinrichtung 13 ebenso bei der Sperreinrichtung durch das Formschlusselement 4 ist es vorteilhaft, wenn der Prothesennutzer ein haptisches Feedback bekommt, um auf mehrfache Art und Weise den Prothesennutzer darauf aufmerksam zu machen, dass zwischen einem entriegelten und einem verriegelten Zustand gewechselt wurde.

Statt eines halbierten Bolzens kann das Formschlusselement auch als schwenkbare Leiste mit einem entsprechenden Betätigungshebel 24 und einer Federvorbelastung ausgebildet sein. Die Ausnehmung 40 ist dann vorzugsweise nicht mehr als Halbkreis, sondern als eine dem Querschnitt der Leiste korrespondierende Ausnehmung ausgebildet.

Das Prothesenkniegelenk 1 ist ein sogenanntes monozentrisches Modular-Leichtkniegelenk mit einer integrierten Miniaturhydraulik 5. Die Hydraulik 5 wird zur Steuerung der Schwungphase verwendet und erzeugt dabei dynamische Bewegungswiderstände, die das Gangbild optimieren, indem sie ein zu weites Durchschwingen in der Beugung oder ein zu hartes Anschlagen in der Streckung verhindern. Die Sperrung des Prothesenkniegelenkes ist mit dem Formschlusselement 4 über den Hebel 24 oder einen anderen Aktuator bedienbar. Der Hebel 24, das Formschlusselement 4 oder der Aktuator können über eine Rändelschraube, eine Stellschraube oder einen axial verschiebbaren Bolzen in den jeweiligen Endlagen fixiert sein, sowohl in der Verriegelungsstellung als auch in der Freigabestellung. Die beiden Stellungen mit den jeweiligen Ausnehmungen 7 sind in der Figur 4 dargestellt. Dadurch ist es möglich, sowohl in der Verriegelungsstellung als auch in der Freigabestellung das Formschlusselement 4 zu fixieren.

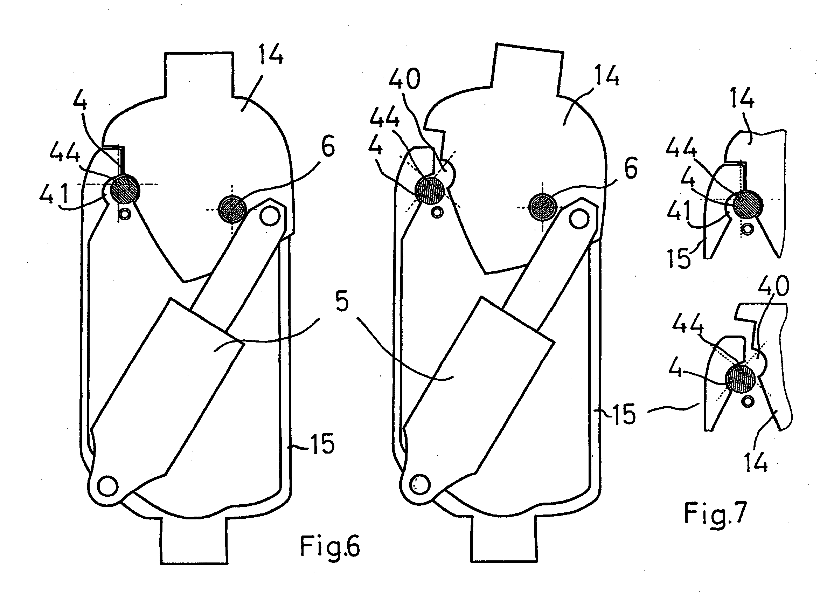

In der Figur 6 sind zwei Darstellungen eines Prothesenkniegelenkes 1 in geschlossener und geöffneter Position dargestellt. In der linken Darstellung ist das erste Teil 14 oder Oberteil in einer Verriegelungsstellung gezeigt. Das Oberteil 14 ist gelenkig um die Schwenkachse 6 gelagert, die Schwenkbewegung kann durch die Dämpfereinrichtung 5 beeinflusst werden. Das Formschlusselement 4 ist schwenkbar um eine Schwenkachse 44 an dem Unterteil 15 gelagert. Anders als bei der Ausführungsform gemäß der Figuren 1 und 2 ist das Formschlusselement 4 um eine Schwenkachse 44 gelagert, die exzentrisch zu dem Formschlusselement 4 angeordnet ist. Das Formschlusselement 4 kann daher im Bereich des Eingriffs in die Ausnehmung 40 auch einen Vollkreisquerschnitt aufweisen, da bei einem Verdrehen des Formschlusselementes 4 aus der Verriegelungsstellung in die Freigabestellung das Formschlusselement aus der Ausnehmung 40 herausschwenken kann und außerhalb des Verschwenkweges, der von dem Oberteil 14 beim Verschwenken zurückgelegt wird, verbleiben kann.

Um ein Herausschwenken des Formschlusselementes 4 aus der Ausnehmung 40 zu ermöglichen, ist eine Aussparung 41 an dem Unterteil 15 vorgesehen, in die das Formschlusselement 4 hineinschwenken kann.

In der rechten Darstellung ist das Prothesenkniegelenk 1 in einer Freigabestellung gezeigt, bei der das Formschlusselement 4 aus der Ausnehmung 40 in dem Oberteil 14 herausgeschwenkt und in die Aussparung 41 in dem Unterteil 14 hineingeschwenkt ist. In der Freigabestellung kann das Oberteil 14 an dem Formschlusselement 4 vorbeigeschwenkt werden, ohne dass eine Blockierung stattfindet.

Durch eine geeignete Wahl der Lage der Schwenkachse 44 ist es möglich, unterschiedliche Vorzugslagen zu kreieren. So ist es beispielsweise möglich, dass ohne Federvorspannung die Position der Schwenkachse 44 so gewählt wird, dass zunächst stets eine Freigabeposition, wie sie in der rechten Darstellung der Figur 6 dargestellt ist, eingenommen wird, alternativ dazu kann eine Ausrichtung in Richtung auf die Ausnehmung 40 des Oberteils 14 erfolgen, so dass ohne eine separate Entriegelungsbewegung das Prothesenkniegelenk 1 grundsätzlich verriegelt ist.

Durch die Kreisform sowohl des Formschlusselementes 4 als auch der Ausnehmung 40 und der Aussparung 41 wird wirksam ein Herausdrücken des Formschlusselementes 4 aus der Verriegelungsstellung verhindert, wenn das Oberteil 14 um die Schwenkachse 6 in Flexionsrichtung bewegt wird. In der Figur 7 sind die verschiedenen Stellungen des Formschlusselementes in Detaildarstellung gezeigt. In der oberen Darstellung befindet sich das Prothesenkniegelenk in einer Verriegelungsstellung, das Formschlusselement 4 ist um seine Schwenkachse 44 herum in Richtung auf das Oberteil 14 verschwenkt, so dass eine Schwenkbewegung des Oberteils 14 nicht erfolgen kann. In der unteren Darstellung ist das Formschlusselement 4 um die an dem Unterteil 15 gelagerte Schwenkachse 14 herum aus der Ausnehmung 40 herausgeschwenkt, so dass das Oberteil 14 an dem Unterteil 15 vorbeischwenken kann, so dass das Prothesenkniegelenk 1 gebeugt werden kann. Alternativ zu einer kreisrunden Querschnittsausgestaltung kann auch eine elliptische oder eine eckige Kontur des Formschlusselementes 4 vorgesehen sein.

## Patentansprüche

1. Gelenkeinrichtung einer Knieorthese oder Knieprothese mit einem ersten Teil (14) und einem relativ dazu verschwenkbaren zweiten Teil (15) und Anschlussmitteln (2) zum Befestigen der Gelenkeinrichtung (1) an einem Nutzer der Knieorthese oder Knieprothese sowie einer Verriegelungseinrichtung (4, 40), mit der eine Verschwenkung der beiden Teile (14, 15) zueinander in Flexionsrichtung durch ein an dem zweiten Teil (15) verlagerbar angeordnetes Formschlusselement (4) verhindert wird, das im Verriegelungszustand formschlüssig in eine dem ersten Teil (14) zugeordnete Ausnehmung (40) eingreift, wobei das Formschlusselement (4) drehbar an dem zweiten Teil (15) gelagert ist und eine Kontur aufweist, die in der Verriegelungsstellung in die Ausnehmung (40) hineinragt und in der Freigabestellung ein Vorbeischwenken des ersten Teils (14) an der Lagerstelle des Formschlusselementes (4) ermöglicht, **dadurch gekennzeichnet, dass** das Formschlusselement (4) als Bolzen mit einem runden oder ovalen Querschnitt mit einer Abflachung (9) zur Ausbildung eines Durchganges für den ersten Teil (14) ausgebildet ist.

2. Gelenkeinrichtung nach Anspruch1 **dadurch gekennzeichnet, dass** das Formschlusselement (4) in Richtung auf die Verriegelungsstellung federbelastet vorgespannt ist.

3. Gelenkeinrichtung nach Anspruch 1 oder 2, **dadurch gekennzeichnet, dass** das Formschlusselement (4) exzentrisch an dem zweiten Teil (15) gelagert ist.

4. Gelenkeinrichtung nach einem der voranstehenden Ansprüche, **dadurch gekennzeichnet, dass** die Gelenkeinrichtung (1) als monozentrische Gelenkeinrichtung ausgebildet ist.

5. Gelenkeinrichtung nach einem der voranstehenden Ansprüche, **dadurch gekennzeichnet, dass** die Gelenkeinrichtung (1) wasserdicht gegenüber der Umgebung abgedichtet ist.

6. Gelenkeinrichtung nach einem der voranstehenden Ansprüche, **dadurch gekennzeichnet, dass** das Formschlusselement (4) gegen eine Verdrehung gesichert an dem zweiten Teil (15) gelagert ist

7. Gelenkeinrichtung nach einem der voranstehenden Ansprüche, **dadurch gekennzeichnet, dass** eine federbelastete Fixiereinrichtung (7) eine formschlüssige Sicherung des Formschlusselementes (4) ausbildet.

8. Gelenkeinrichtung nach einem der voranstehenden Ansprüche, **dadurch gekennzeichnet, dass** das Formschlusselement (4) entlang seiner Drehachse verschiebbar ausgebildet ist.

9. Gelenkeinrichtung nach einem der voranstehenden Ansprüche, **dadurch gekennzeichnet, dass** zwischen dem ersten Teil (14) und dem zweiten Teil (15) ein Dämpfer (5) angeordnet ist.

10. Gelenkeinrichtung nach einem der voranstehenden Ansprüche, **dadurch gekennzeichnet, dass** die Ausnehmung (40) an dem ersten Teil (14) so angeordnet ist, dass die Verriegelungsstellung in einer gestreckten Stellung der Gelenkeinrichtung (1) vorliegt.

11. Gelenkeinrichtung nach einem der voranstehenden Ansprüche, **dadurch gekennzeichnet, dass** dem Formschlusselement (4) Drehanschläge zugeordnet sind.

12. Gelenkeinrichtung nach einem der voranstehenden Ansprüche, **dadurch gekennzeichnet, dass** der Fixiereinrichtung (7, 10) ein fernbedienbarer Aktuator (11) zugeordnet ist.

## Claims

1. A joint mechanism of a knee orthosis or knee prosthesis having a first part (14) and a second part (15), which is pivotable in relation thereto, and connection means (2) for fastening the joint mechanism (1) on a user of the knee orthosis or knee prosthesis, as well as having a locking mechanism (4, 40), by way of which a pivoting of the two parts (14, 15) relative to one another in the direction of flexion is prevented by means of a positive locking element (4), which is arranged so as to be displaceable on the second part (15) and, in the locking state, engages in a positive locking manner in a recess (40) associated with the first part (14), wherein the positive locking element (4) is mounted so as to be rotatable on the second part (15) and has a contour which, in the locking position, projects into the recess (40) and in the release position enables the first part (14) to pivot past the bearing of the positive locking element (4), **characterized in that** the positive locking element (4) is realized as a bolt with a round or oval cross section with a flattening (9) to realize a passage for the first part (14).

2. The joint mechanism as claimed in claim 1, **characterized in that** the positive locking element (4) is prestressed in a spring-loaded manner in the directing of the locking position.

3. The joint mechanism as claimed in claim 1 or 2, **characterized in that** the positive locking element (4) is mounted in an eccentric manner on the second part (15).

4. The joint mechanism as claimed in one of the preceding claims, **characterized in that** the joint mechanism (1) is realized as a monocentric joint mechanism.

5. The joint mechanism as claimed in one of the preceding claims, **characterized in that** the joint mechanism (1) is sealed in a water-tight manner in relation to the surrounding area.

6. The joint mechanism as claimed in one of the preceding claims, **characterized in that** the positive locking element (4) is mounted on the second part (15) so as to be secured against rotation.

7. The joint mechanism as claimed in one of the preceding claims, **characterized in that** a spring-loaded fixing device (7) realizes a positive-locking securement of the positive locking element (4).

8. The joint mechanism as claimed in one of the preceding claims, **characterized in that** the positive locking element (4) is realized so as to be displaceable along its axis of rotation.

9. The joint mechanism as claimed in one of the preceding claims, **characterized in that** a damper (5) is arranged between the first part (14) and the second part (15).

10. The joint mechanism as claimed in one of the preceding claims, **characterized in that** the recess (40) is arranged on the first part (14) such that the locking position is present in an extended position of the joint mechanism (1).

11. The joint mechanism as claimed in one of the preceding claims, **characterized in that** rotational stops are associated with the positive locking element (4).

12. The joint mechanism as claimed in one of the preceding claims, **characterized in that** a remotely-controllable actuator (11) is associated with the fixing device (7, 10).

## Revendications

1. Mécanisme d'articulation d'une orthèse de genou ou d'une prothèse de genou comprenant une première partie (14) et une seconde partie (15) capable de pivoter par rapport à celle-ci, et des moyens de raccordement (2) pour fixer le mécanisme d'articulation (1) sur un utilisateur de l'orthèse de genou ou de la prothèse de genou, ainsi qu'un système de verrouillage (4, 40) au moyen duquel un pivotement des deux parties (14, 15) l'une par rapport à l'autre en direction de flexion est empêché au moyen d'un élément à coopération de formes (4) agencé déplaçable sur la seconde partie (15), élément qui, dans la situation de verrouillage, s'engage par coopération de formes dans un évidement (40) associé à la première partie (14), dans lequel l'élément à coopération de formes (4) est monté rotatif sur la seconde partie (15) et présente un contour qui, dans la position de verrouillage, pénètre dans l'évidement (40) et qui, dans la position de libération, permet un pivotement de la première partie (14) le long de l'emplacement de montage de l'élément à coopération de formes (4), **caractérisé en ce que** l'élément à coopération de formes (4) est réalisé comme un goujon avec une section ronde ou ovale présentant un méplat (9) pour réaliser une traversée pour la première partie (14).

2. Mécanisme d'articulation selon la revendication 1, **caractérisé en ce que** l'élément à coopération de formes (4) est précontraint en direction de la position de verrouillage sous la charge d'un ressort.

3. Mécanisme d'articulation selon la revendication 1 ou 2, **caractérisé en ce que** l'élément à coopération de formes (4) est monté de manière excentrique sur la seconde partie (15).

4. Mécanisme d'articulation selon l'une des revendications précédentes, **caractérisé en ce que** le mécanisme d'articulation (1) est réalisé sous forme de mécanisme d'articulation mono-centré.

5. Mécanisme d'articulation selon l'une des revendications précédentes, **caractérisé en ce que** le mécanisme d'articulation (1) est étanché de manière étanche à l'eau par rapport à l'environnement.

6. Mécanisme d'articulation selon l'une des revendications précédentes, **caractérisé en ce que** l'élément à coopération de formes (4) est monté de façon bloquée sur la seconde partie (15) à l'encontre d'une rotation.

7. Mécanisme d'articulation selon l'une des revendications précédentes, **caractérisé en ce qu'**un système de fixation (7) chargé par ressort réalise un blocage à coopération de formes de l'élément à coopération de formes (4).

8. Mécanisme d'articulation selon l'une des revendications précédentes, **caractérisé en ce que** l'élément à coopération de formes (4) est réalisé déplaçable le long de son axe de rotation.

9. Mécanisme d'articulation selon l'une des revendications précédentes, **caractérisé en ce qu'**un amortisseur (5) est agencé entre la première partie (14) et la seconde partie (15).

10. Mécanisme d'articulation selon l'une des revendications précédentes, **caractérisé en ce que** l'évidement (40) est agencé sur la première partie (14) de telle façon que la position de verrouillage se présente dans une position en extension du mécanisme d'articulation (1).

11. Mécanisme d'articulation selon l'une des revendications précédentes, **caractérisé en ce que** des butées de rotation sont associées à l'élément à coopération de formes (4).

12. Mécanisme d'articulation selon l'une des revendications précédentes, **caractérisé en ce qu'**un actionneur (11) capable d'être télécommandé est associé au système de fixation (7, 10).
